# EUROPEAN PATENT APPLICATION

(11) **EP 0 547 687 A1**
(43) Date of publication of application: **23.06.1993**
(21) Application number: 92203853.4
(22) Date of filing: 10.12.1992
(51) Int. Cl.: C07J 73/00, A61K 31/58

(54) **17B-N-Substituted adamantyl/norbonanyl carbamoyl-4-aza-5a-and-rost-1-en-3-ones and androstan-3 ones**

(30) Priority: 18.12.1991 US 809995
(71) Applicant: MERCK & CO. INC., Rahway New Jersey 07065-0900 (US)
(72) Inventor: Steinberg, Nathan G., Clark, NJ 07066 (US); Salzmann, Thomas N., North Plainfield, NJ 07062 (US); Rasmusson, Gary H., Watchung, NJ 07060 (US); Patel, Gool F., Millington, NJ 07060 (US)
(74) Representative: Thompson, John Dr.

(57) **Abstract**

17β-N-substituted adamantyl/norbornanyl carbamoyl-4-aza-5α-androst-1-en-3-ones and androstan-3-ones as 5α-reductase inhibitors of the formula:

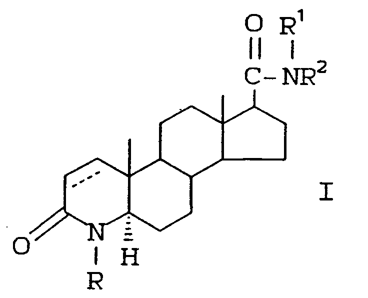

## Description

### BACKGROUND OF THE INVENTION

The present invention is concerned with novel 17β-N-substituted adamantyl or norbornanyl carbamoyl-4-aza-5α-androst-1-en-3-ones and androstan-3-ones compounds and the use of such compounds as testosterone-5α-reductase inhibitors.

### DESCRIPTION OF THE PRIOR ART

It is well known in the art that certain undesirable physiological manifestations, such as acne vulgaris, seborrhea, female hirsutism, and male pattern baldness and benign prostatic hypertrophy, are the result of hyperandrogenic stimulation caused by an excessive accumulation of testosterone or similar androgenic hormones in the metabolic system. Early attempts to provide a chemotherapeutic agent to counter the undesirable results of hyperandrogenicity resulted in the discovery of several steroidal anti-androgens having undesirable hormonal activities of their own. The estrogens, for example, not only counteract the effect of the androgens but have a feminizing effect as well. Non-steroidal anti-androgens have also been developed, for example, 4'-nitro-3'-trifluoromethylisobutyranilide. See Neri et al., Endo., Vol. 91, No. 2 (1972). However, these products, though devoid of hormonal effects, are peripherally active, competing with the natural androgens for receptor sites, and hence have a tendency to feminize a male host or the male fetus of a female host.

It more recently became known in the art that the principal mediator of androgenic activity in some target organs is 5α-dihydrotestosterone, and that it is formed locally in the target organ by the action of testosterone-5α-reductase. It therefore has been postulated and demonstrated that inhibitors of testosterone-5α-reductase will serve to prevent or lessen symptoms of hyperandrogenic stimulation. Nayfeh et al., Steroids, 14, 269 (1969) demonstrated in vitro that methyl 4-androsten-3-one-17β-carboxylate was a testosterone-5α-reductase inhibitor. Then Voigt and Hsia, Endocrinology, 92, 1216 (1973), Canadian Pat. No. 970,692, demonstrated that the above ester and the parent free acid, 4-androsten-3-one-17β-carboxylic acid are both active inhibitors of rat prostatic testosterone-5α-reductase in vitro. They further demonstrated that topical application of either testosterone or 5α-dihydrotesterone caused enlargement of the female hamster flank organ, an androgen dependent sebaceous structure. However, concomitant administration of 4-androsten-3-one-17β-carboxylic acid or its methyl ester inhibited the response elicited by testosterone but did not inhibit the response elicited by 5α-dihydrotestosterone. These results were interpreted as indicating that the compounds were antiandrogenlc by virtue of their ability to inhibit the enzyme, testosterone-5α-reductase.

A number of 4-aza steroid compounds are known. See, for example, U.S. Pat. Nos. 2,227,876; 3,239,417; 3,264,301; and 3,285,918; French Pat. No. 1,465,544; Doorenbos and Solomons, J. Pharm. Sci. 62, 4, pp. 638-640 (1973); Doorenbos and Brown, J. Pharm. Sci., 60 8, pp. 1234-1235 (1971); and Doorenbos and Kim, J. Pharm. Sci. 63, 4, pp. 620-622 (1974).

In addition, U.S. Patents 4,377,584, 4,220,775, 4,859,681, 4,760,071 and the articles J. Med. Chem. 27, p. 1690-1701 (1984) and J. Med. Chem. 29, 2298-2315 (1986) of Rasmusson et al., U.S. Patent 4,845,104 to Carlin et al. and U.S. Patent 4,732,897 to Cainelli et al. describe 4-aza-17β-substituted-5α-androstan-3-ones which are said to be useful in the treatment of DHT-related hyperandrogenic conditions.

Further described in the field are the following two prior art references:
Proc. Natl. Acad. Sci, USA, Vol. 87, pp. 3640-3645, May 1990 by S. Andersson and D.W. Russell which describes structural and biochemical properties of cloned and expressed human and rat steroid 5-alpha reductases; and
Nature, Vol 354, Nov. 1991, pp 159-161 by S. Andersson, et al., which describes the isolation of a second human enzyme, 5-alpha reductase 2, and the effect of a deletion in this gene in male pseudohermaphroditism.

As described in the above papers by Andersson, Russell et al., two human isozymes of 5-alpha reductase were obtained from a human prostatic DNA library. These enzymes have been identified as 5-alpha reductase enzymes 1 and 2. Both enzymes can produce DHT and can contribute to elevated DHT levels which are implicated in the hyperandrogenic conditions of alopecia, male pattern baldness, acne, benign prostatic hyperplasia, and prostatic cancer. PROSCAR® (Merck & Co. Inc., being 17 beta (N-t-butyl-carbamoyl) androst-1-en-3-one, see USP 4,760,071) has been shown to be active against 5-alpha reductase enzyme 2, but is poorly effective against 5 alpha reductase 1. The compound 4MA (17 beta-(N-,N-diethyl-carbamoyl)androstane-3-one) has been shown to be more effective against 5-alpha reductase enzyme 1 and is apparently also effective against 5-alpha reductase enzyme 2. However, 4MA suffers the severe disadvantage of being hepatotoxic in dogs. What is desired in the art is a compound which acts as a dual inhibitor againt both 5-alpha reductase enzymes 1 and 2 to more effectively treat the hyperandrogenic conditions of alopecia, male pattern baldness, acne, female hirsutism, benign prostatic hyperplasia, and prostatic carcinoma and which does not display undesired hepatotoxicity.

### SUMMARY OF THE INVENTION

The present invention relates to novel 17β-N-(substituted) adamantyl and norbornanyl carbamoyl-4-aza-5α-androsten-1-en-3-ones and androstan-3-ones compounds, processes for their preparation, pharmaceutical formulations comprising these novel compounds as active ingredients and methods of dual inhibition of both 5α-reductase enzymes 1 and 2 and of treating hyperandrogenic conditions involving the 5α-reductase enzymes 1 and 2 with the novel compounds disclosed herein and their pharmaceutical formulations.

In accordance with this invention, there is provided compounds of the formula: 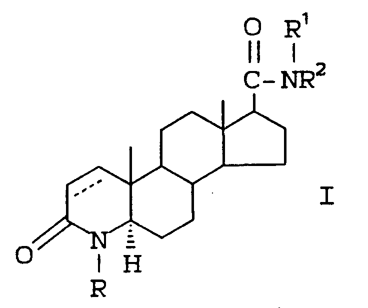
wherein
- R and R¹: are independently selected from hydrogen, methyl or ethyl;
- R²: is a hydrocarbon radical selected from substituted or unsubstituted 1- or 2-adamantyl, 1- or 2-adamantylmethyl, or 1-, 2- (exo or endo) or 7-norbornanyl, 1-, 2- or 7-norbornanylmethyl, provided that when R¹ is hydrogen, then R² does not represent substituted or unsubstituted 1- or 2-adamantyl, or 1- or 2-norbornanyl;
the dotted line represents a double bond which can be present, and pharmaceutically acceptable salts or esters thereof.

### BRIEF DESCRIPTION OF THE INVENTION AND PREFERRED EMBODIMENTS

Representative compounds of the present invention include the following:
17β-(N-(3-methyl)-1-adamantylcarbamoyl)-4-aza-4-methyl-5α-androstan-3-one;
17β-(N-exo-2-norbornanylcarbamoyl)-4-aza-4-methyl-5α-androst-1-en-3-one;
17β-(N-1-adamantylmethylcarbamoyl)-4-aza-5α-androst-1-en-3-one;
17β-(N-2-adamantylcarbamoyl)-4-aza-5α-androstan-3-one;
17β-(N-methyl-N-2-adamantylcarbamoyl)-4-aza-4-methyl-androstan-3-one;
17β-(N-2-adamantylcarbamoyl)-4-methyl-4-aza-5α-androstan-3-one; and
17β-(N-methyl-N-2-adamantyl)carbamoyl-4-methyl-4-aza-androst-1-en-3-one.
the corresponding compounds wherein the 4-aza substituent is substituted in each of the above named compounds with a hydrogen, methyl or an ethyl radical, to form a different N-substituent, and wherein a double bond can be optionally present as indicated by the dotted line in position 1.

The 1-, 2-adamantyl or 1-, 2-norbornanyl moieties can be substituted with one or more substituents of the following: C₁-C₄ linear/branched alkyl, including methyl, ethyl, isopropyl, n-butyl; nitro; oxo; C₇-C₉ aralkyl, including benzyl; (CH₂)n COOR where n is 0-2 and R is H or C₁-C₄ linear/branched alkyl including methyl, ethyl; CH₂OH; OH; OR where R is C₁-C₄ linear/branched alkyl including methyl, ethyl; halo, including fluoro, bromo, iodo; COOH; COOR, where R is linear/branched C₁-C₄ alkyl; -CONH₂; CH₂NH₂; CH₂NHCOR where R is C₁-C₄ linear/branched alkyl including methyl, ethyl; phenyl; o, m, p-substituted phenyl including p-nitro, p-amino and p-sulfo; or cyano. The amino group of the adamantyl or norbornanyl moiety can also be substituted as R¹ with methyl and ethyl, as well as hydrogen.

Also included within the scope of this invention are pharmaceutically acceptable salts or esters, where a basic or acidic group is present on the adamantyl or norbornanyl moiety. When an acidic substituent is present, i.e. -COOH, there can be formed the ammonium, sodium, potassium, calcium salt, and the like, for use as the dosage form.

Where a basic group is present, i.e. amino, acidic salts, i.e. hydrochloride, hydrobromide, acetate, pamoate, and the like, can be used as the dosage form.

Also, in the case of the -COOH group being present, pharmaceutically acceptable esters can be employed, e.g. acetate, maleate, pivaloyloxymethyl, and the like, and those esters known in the art for modifying solubility or hydrolysis characteristics for use as sustained release or prodrug formulations.

Representative examples include for R² (where AD is adamantyl):
3, 5, 7-trinitro-1-AD; 4-oxo-1-AD; 1-benzyl-1-AD; 4,4-dimethyl-1-Ad; 3,7-dimethyl-5-carboxymethyl-1-AD; 3-carboxymethyl-1-AD; 3-chloro-1-AD; 1,3-dihydroxy-6,6-dimethyl-2-AD; 3-chloro-1-AD; 4-carbethoxy-2-AD; 4-carboxy-2-AD; 3-isopropyl-1-AD; 3-n-butyl-1-AD; 3-propyl-1-AD; 3-,5-diethyl-1-AD; 3-hydroxymethyl-1-AD; 2-carboxy-1-AD; 3-methyl-1-AD; 5-hydroxy-2-AD; 2-hydroxy-1-AD; 1-aminomethyl-1-hydroxy-2-AD; 2-oxo-1-AD; 2-phenyl-2-AD; 1-amino-methyl-2-AD; 1-carboxy-2-AD; 1-aminocarbonyl-2-AD; 3-hydroxy-5,7-dimethyl-1-AD; 4-fluoro-1-AD; 3-fluoro-1-AD; 4-hydroxy-2-AD; 3-phenyl-1-AD; 3-(p-aminophenyl)-1-AD; 3-(p-nitrophenyl)-1-AD; 3-methyl-5-hydroxymethyl-1-AD; 3,5-dimethyl-4-hydroxy-1-AD; 2-hydroxymethyl-2-AD; 3-(p-sulfophenyl)-1-AD; 3-methyl-5-ethyl-1-AD; 2-carboxy-2-AD; 3,5-7-trimethyl-1-AD; 4-iodo-2-AD; 4-bromo-2-AD; 4-chloro-2-AD; 1-acetylaminomethyl-2-AD; 1-carboxymethyl-2-AD; 1-methyl-2-AD; 1-aminocarboxylmethyl-2-AD; 1-aminocarboxyl-1-AD; 2-cyano-2-AD; 3,5-dimethyl-7-ethyl-1-AD; 4-hydroxy-1-AD; 1-hydroxy-2-AD; 5-carboxy-3-methyl-1-AD; 3,5-dimethyl-7-carboxy-1-AD; 3-carboxy-1-AD; 3-hydroxy-1-AD; and the like.

Representative examples include for R² as substituted norbornanyl moieties are (where NB is norbornanyl):
2-NB; 1,7,7-trimethyl-4-phenyl-2-NB; 3-carboxy-2-NB; 3-phenyl-2-carboxy-2-NB; 2-cyano-3-phenyl-2-NB; 3-hydroxy-4,7,7-trimethyl-2-NB; 6-hydroxymethyl-2-NB; 5-cyano-2-NB; 3-allyl-2-NB; 1-NB; 7,7-dimethyl-1-hydroxymethyl-2-NB; 3-methoxy-4,7,7-trimethyl-2-NB; 3-aminocarbonyl-2-NB; 3-ethoxycarbonyl-2-NB; 3,3-dimethyl-2-NB; 7-oxo-1-NB; 3-phenyl-2-NB; 1-carboxymethyl-7,7-dimethyl-2-NB; 1-ethyl-2-NB; 1-methyl-2-NB; 2,2,3,3,5,5,6,6,7,7-decafluoro-1-NB; 3-hydroxy-2-NB; 3-chloro-2-NB; 3-(p-methoxyphenyl)-2-NB; 2,2-dimethyl-3-methylene-7-NB; 3-oxo-2-NB; 1-methoxy-2-NB; 7-NB; 3-isopropyl-2-NB; 2-bromo-1-NB; 3-chloro-1-NB; and the like.

Procedures for preparing the adamantyl and norbornanyl amines useful in this invention, including the above, are well known in the art.

The novel compounds of formula I of the present invention can be prepared by a method starting with the known steroid ester (II) of the formula: 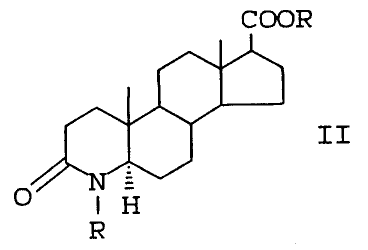
17β-(carbomethoxy)-4-aza-5-α-androstan-3-ones which includes the stages of optionally 1) dehydrogenating said starting material to produce the corresponding compound containing a double-bond in the 1,2-position of the A-ring, 2) converting the 17-carbomethoxy substituent into an N-monosubstituted adamantyl-carbamoyl substituent and, if desired, 3) alkylating the A-ring nitrogen to introduce a N-methyl or N-ethyl substituent into the A ring 4-position. For the dehydrogenatin step, it is preferable that the 4-aza nitrogen be unsubstituted. The alternate pathways can consist of one or more discrete chemical steps and if desired can take place before step (1) or following step (1) or step (3).

In accordance with the process of the present invention (see flow sheet), the products of our invention are formed by optionally: (1) heating a 17β-alkoxycarbonyl-4-aza-5α-androstan-3-ones, compound III, (prepared in the literature as described in the reference US Patent 4,377,584) with a dehydrogenating agent such as benzeneseleninic anhydride in a refluxing inert solvent, e.g. chlorobenzene, to form a 17β-alkoxycarbonyl-4-aza-5α-androst-1-ene-3-one IV (alternately, the dichlorodicyanobenzoquinone process of Dolling, et al., JACS 1988, Vol. 110, pp. 3318-3319, can be used); (2) the formed 5α-androst-1-en-3-one compound from Step 1 can be reacted with, e.g. sodium hydride under anhydrous conditions in a neutral solvent such as dimethylformamide; (3) contacting the resulting reaction mixture with an alkyl (methyl or ethyl) iodide to form the corresponding 17-β-alkoxy-adamantyl-carbamoyl-4-alkyl-4-aza-5α-androst-1-en-3-one V; (4) subsequently hydrolyzing said 17β-alkoxycarbonyl-4-alkyl-4-aza-5α-androst-1-en-3-one with a strong base, such as aqueous methanolic potassium hydroxide at the reflux temperature, followed by acidification and isolation of the resulting steroidal acid to yield 17β-carboxy 4-alkyl-4-aza-5α-androst-1-en-3-one VI; (5) said steroidal acid can be then converted to its corresponding 2-pyridylthio ester by refluxing with triphenyl phosphine and 2,2'-dipyridyl disulfide in an inert solvent such as toluene and the resulting product 17β-(2-pyridylthiocarbonyl)-4-alkyl-4-aza-5α-androst-1-en-3-one VII can be isolated by chromatography on e.g. silica gel; and (6) said pyridylthio ester can be thenreacted with 1-adamantyl-, 2-adamantylamine or norbornanylamine in an inert solvent e.g. tetrahydrofuran, to form the desired product 17β-N-adamantyl-carbamoyl-4-alkyl-4-aza-5α-androst-1-en-3-one VIII which can be isolated by chromatography e.g. on silica gel. When the previous reaction is carried out in the absence of first forming the double bond at position 1, the corresponding 17β-(N-adamantyl-carbamoyl)-4-alkyl-4-aza-5α-androstan-3-one (or N-norbornanyl carbamoyl compound) is prepared.

In accordance with an alternate process of our invention the corresponding N-unsubstituted-17β(N-adamantyl-carbamoyl)-4-aza-5α-androst-1-en-3-one XIV is readily prepared from the 17β (alkoxycarbonyl)-4-aza-5α-androstone-3-one IV by repeating the above series of reaction steps but omitting the alkylation Step 2 herein above, i.e. treatment of the 4-aza-5-α-androst-1-en-3-one with e.g. sodium amide followed by methyl or ethyl iodide via intermediates XII and XIII.

In accordance with a further alternate process of preparing the compounds of our invention having only hydrogen as the sole substituent on the ring A - nitrogen, the double bond in the A ring is introduced as the last step of the process. Thus, a 17β-alkoxycarbonyl 4-aza-5α-androstan-3-one III is hydrolyzed to the corresponding steroidal acid IX 17β-carboxy-4-aza-5α-androstan-3-one which in turn is converted to the corresponding pyridylthio ester, 17β (2-pyridylthiocarbonyl)-4-aza-5α-androstan-3-one, X followed by treatment of the ester with an amine of formula R²-NH₂ wherein R² is as defined hereinabove as 1- or 2-adamantyl or 1-, 2-, or 7-norbornanyl to form a 17β (N-adamantyl-carbamoyl)-4-aza-5α-androstone-3-one XI which is dehydrogenated as previously described to produce compound XIV, 17β-(N-adamantyl-carbamoyl)-4-aza-androst-1-en-3-one or corresponding norbornanyl derivative.

In another alternate method of introducing the 17β-(N-adamantyl-carbamoyl)substituent into a 17β-carboxy androstane compound of formula VI, XII or IX, each is treated in a manner similar to the procedure described in Steroids, Vol. 35 #3, March 1980, p. 1-7 with dicyclohexylcarbodiimide and 1-hydroxybenzo-triazole to form the 17β-(1-benzotriazoloxycarbonyl)-4-aza-5α-androst-1-en-3-one, VII, XIII or compound X, wherein the substituent X is benzotriazoloxy group.

The above reactions are schematically represented in the following flowsheet.

### Flowsheet

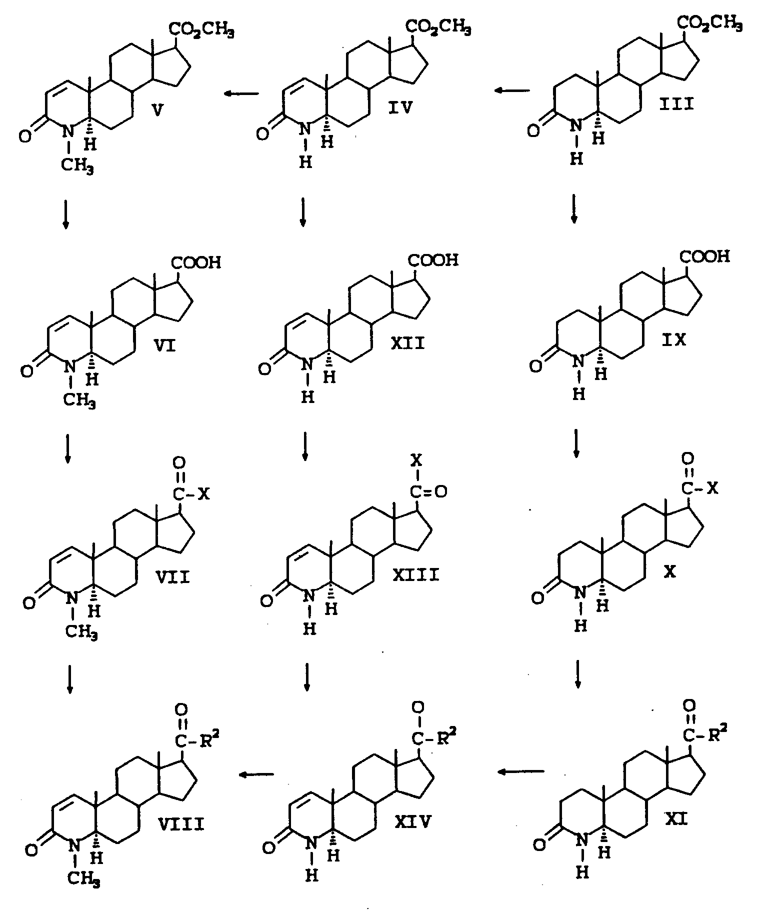

- X: is 2-pyridylthio or 1-benzotriazoloxy.
- R²: is 1- or 2-adamantyl or norbornanyl.

The compounds of the present invention, prepared in accordance with the method described above, are, as already described, potent agents by virtue of their ability to specifically inhibit different forms of 5α-reductases.

Accordingly, the present invention is particularly concerned with providing a method of treating the hyperandrogenic conditions of acne vulgaris, seborrhea, and female hirsutism by topical administration, and a method of treating all of the above conditions as well as benign prostatic hypertrophy, by oral or parenteral administration, of the novel compounds of the present invention.

The present invention is thus also concerned with providing suitable topical, oral and parenteral pharmaceutical formulations for use in the novel methods of treatment of the present invention.

The compositions containing the compounds of the present invention as the active ingredient for use in the treatment of benign prostatic hypertrophy can be administered in a wide variety of therapeutic dosage forms in conventional vehicles for systemic administration, as, for example, by oral administration in the form of tablets, capsules, solutions, or suspensions, of by intravenous injection. The daily dosage of the products may be varied over a wide range varying from 0.05 to 2,000 mg. The compositions are preferably provided in the form of scored tablets containing 0.01, 0.1, 1, 5, 10, 25, 50, 100, 150, 250, and 500 milligrams of the active ingredient for the symptomatic adjustment of the dosage to the patient to be treated. An effective amount of the drug is ordinarily supplied at a dosage level of from about 0.01 mg. to about 50 mg./kg. of body weight per day. Preferably the range is from about 0.02 mg. to 75 mg./kgs. of body weight per day. This translates to a dosage of about 1 to 2000 mg/day, preferably about 1 to 20 mg/day. These dosages are well below the toxic dose of the product. Capsules containing the product of this invention can be prepared by mixing an active compound of the present invention with lactose and magnesium stearate, calcium stearate, starch, talc, or other carriers, and placing the mixture in gelatin capsule. Tablets may be prepared by mixing the active ingredient with conventional tableting ingredients such as calcium phosphate, lactose, corn starch or magnesium stearate. The liquid forms in suitably flavored suspending or dispersing agents such as the synthetic and natural gums, for example, tragacanth, acacia, methylcellulose and the like. Other dispersing agents which may be employed include glycerin and the like. For parenteral administration, sterile suspensions and solutions are desired. Isotonic preparations which generally contain suitable preservative are employed when intravenous administration is desired.

For the treatment of acne vulgaris, seborrhea, female hirsutism, the compounds of the present invention are administered in the formula of pharmaceutical composition comprising the active compound in combination with a pharmacologically acceptable carrier adapted for topical administration. These topical pharmaceutical compositions may be in the form of a cream, ointment, gel or aerosol formulation adapted for application to the skin. These topical pharmaceutical compositions containing the compounds of the present invention ordinarily include about 0.01% to 15%, preferably about 1%, of the active compound, in admixture with about 9.9% of vehicle.

The method of preparing the novel 17β-N-substituted adamantyl-carbamoyl compounds of the present invention, already described above in general terms, may be further illustrated by the following examples.

### EXAMPLE 1

### Methyl 3-oxo-4-aza-5α-androst-1-ene-17β-carboxylate

A suspension of 83.7 g of methyl 3-oxo-4-aza-5α-androstane-17-carboxylate (III) (Rasmusson, et al. U.S. Patent 4,760,071.) and 126.5 g of benzene-seleninic anhydride in 2.09 l of chlorobenzene was heated at reflux for 2 hours. The reflux condenser was switched to a distillation head and the mixture was distilled slowly to remove water that had formed in the reaction (2 hours). The solution was evaporated to leave 198 g of wet residue. The residue as a solution in dichloromethane was washed with saturated aqueous NaHCO₃ solution and saturated NaCl solution, then dried and evaporated to leave 172.4 g. This material was chromatographed on 2.56 kg of silica gel eluting first with dichloromethane (5 liters) and then with 4:1 dichloromethane acetone. The desired product was eluted with 8 liters of the above-mixed solvent and evaporated in vacuo to yield 53.4 g. It was washed with diethyl ether and dried to leave 49.5 g of the unsaturated title product, m.p. 278-280°C.

### EXAMPLE 2

### Synthesis of 17β(N-1-adamantyl-carbamoyl)-4-aza-5α-androst-1-en-3-one

100 mg of the 17-methyl ester (0.305 mmoles) from Example 1 was suspended in 3.0 ml of THF (dried over molecular sieves 3A), and then was added 183.0 mg of 1-adamantanamine (1.2 mmoles). The suspension was cooled to 5-10°C and then 590 µl of 2.0 M solution, of EtMgBr in THF was added. The resulting mixture was allowed to stir for 10 minutes, and then refluxed for 1-2 hours under N₂. The mixture was cooled to 0°C and then quenched with saturated solution of NH₄Cl (about 10 ml.). The organic layer was separated and the aqueous layer extracted with three volumes CH₂Cl₂.

The organic layers were combined, washed 2 times with H₂O, twice with saturated sodium chloride, and dried over MgSO₄, filtered and evaporated to dryness in vacuum. Crystallization from EtOAc afforded 75.0 mg of product. Recrystallization from MeOH and drying at 110°C for 2 hours/0.1 mm gave product, mpt. 305-306°C. Molecular weight (by FAB) showed M⁺=451: Calculated =451.

| Anal. Calcd. for C₂₉H₄₂N₂O₂: | | | | | | |
|---|---|---|---|---|---|---|
| | C, | 77.28; | H, | 9.40; | N, | 6.21. |
| Found: | C, | 76.84; | H, | 9.73; | N, | 5.93. |

### EXAMPLE 3

### Synthesis of 17β(N-2-adamantyl-carbamoyl)-4-aza-5α-androst-1-en-3-one

Following the above-described general procedure of Example 2 but utilizing 2-adamantamine (prepared by aqueous neutralization of the hydrochloride and EtOAc extraction and isolation) in place of 1-adamantamine, and carrying out the reflux for 7 hours rather than 1-2 hours, the title compound is prepared, mpt. 284-285°C.

### EXAMPLE 4

### Synthesis of 17β(N-1-adamantylcarbamoyl)-4-aza-5α-androstane-3-one

100.0 mg of the adamantyl derivative produced in Example 2 was dissolved in 5.0 ml of dry THF. 300 mg of 5% Pd/C was added and the mixture was hydrogenated for 6.0 hrs. at R.T. at 40 psi. The mixture was filtered through celite, the cake washed with THF (3 times) and solvent evaporated under vacuum to yield 97.0 mg. of crude above-titled product. NMR showed absence of olefins. The crude material was placed on 15.0 g silica gel column, and eluated with 1:1(CH₂Cl₂: acetone).

Collected fractions afforded a single spot material by TLC weighing 77.98 mg. NMR was in excellent agreement with the proposed structure. Recrystallized from EtOAc to yield 65.59 mg of the above-titled product, mp. 323-324°C.

| Anal. Calcd. for C₂₉H₄₄O₂N₂ 1/4 H₂O: | | | | | | |
|---|---|---|---|---|---|---|
| | C, | 76.18; | H, | 9.81; | N, | 6.13. |
| Found: | C, | 75.91; | H, | 9.97; | N, | 6.06. |

### EXAMPLE 5

### Methyl 3-oxo-4-methyl-4-aza-5α-androst-1-ene-17β-carboxylate

A suspension of 25 g of the product of Example 1 and 3.35 g of sodium hydride in 500 ml of dry dimethylformamide was stirred under nitrogen for 15 minutes. Methyl iodide (15 ml) was added dropwise and the mixture was stirred for 30 minutes at room temperature. Additional (5 ml) methyl iodide was added and the mixture was heated at 50°C for 2 hours. After cooling the mixture was diluted with water to 2 liters. The solid was separated after cooling and amounted to 25.4 g of the above-titled product, m.p. 159-161°C.

### EXAMPLE 6

### S-(2-Pyridyl)-3-oxo-4-methyl-4-aza-5α-androst-1-ene-17β-thiocarboxylate

6(A) A suspension of 25 g of the product of Example 5 in 125 ml of methanol was treated with a solution of KOH (12.5 g) in 12.5 ml of water. After refluxing for 4 hours, the solution was acidified with 6N HCl and then was diluted with water. The crude acid (23.32 g) was separated, dried and had a m.p. 300°C.
6(B) The crude, dry acid (23 g), triphenylphosphine (36.45 g) and 2,2'-dipyridyldisulfide (30.4 g) were suspended in 138 ml of toluene with stirring overnight at room temperature. Next day, crystallization set in. The reaction mixture was filtered, the residue washed with cold toluene, followed with cold anhydrous ether. Dried at 110°C in vacuo to afford 20.4 g of the desired above-titled thiopyridyl ester m.pt. 218-220°C.

### EXAMPLE 7

### Synthesis of 17β(N-1-adamantylcarbamoyl)-4-methyl-4-aza-5α-androst-1-en-3-one

120 mg of the thiopyridyl ester of Example 6 was suspended in 20 ml of dry THF, to the suspension was added 175.0 mg of 1-adamantanamine under N₂. The reaction was carried out at R.T. for 16 hours under N₂. The reaction was monitored by silica gel TLC, using 1:1 acetone: hexane. The product was separated on TLC 20 cm x 20 cm, 1000 µm silica gel plate, eluted with 1:1 (acetone/hexane).
The product was crystallized from ethyl acetate, to give 50.0 mg of pure material m. pt. 202-205°C. Molecular Weight (FAB) showed 465; Calc: 465. Recrystallization afforded 19.14 mg of the above-titled product, m.pt. 202-202.5°C.

| Anal. Calcd for C₃₀H₄₄N₂O₂·H₂0: | | | | | | |
|---|---|---|---|---|---|---|
| | C, | 74.64; | H, | 9.60; | N, | 5.80. |
| Found: | C, | 74.32; | H, | 9.47; | N, | 5.89. |

### EXAMPLE 8

### Hydrolysis of Methyl-3-oxo-4-aza-5α-androstane-17β-carboxylate

The 17β-androstane carboxylate starting material of Example 1 was hydrolyzed with 7% KOH in isopropanol or aqueous methanol, followed by an acidic work-up to give the corresponding 17β carboxylic acid which was utilized in Example 9.

### EXAMPLE 9

### N-(1-adamantyl)-3-oxo-4-aza-5α-androstane-17β-carboxamide

A solution of 5.0 g of the product of Example 8, 3.35 g of dicyclohexylcarbodiimide and 3.18 g of 1-hydroxybenztriazole in 500 ml of dichloromethane was stirred at room temperature overnight. The solid was separated by filtration and the filtrate was treated with 1-adamantamine. This solution stood at room temperature for 64 hours, then filtered, and the solution was washed successively with 10% hydrochloric acid and saturated aqueous sodium chloride. After drying with MgSO₄, it was filtered and concentrated. The crude product was eluted through 240 g of silica gel with 3:7 (acetone-dichloromethane) to give 5.5 g of the above-titled product, m.p. 323-324°C.

### EXAMPLE 10

### Synthesis of Benztriazol-1-yl-3-oxo-4-methyl-4-aza-5α-androstan-17β-carboxylate

A suspension of 83.7 g of methyl-3-oxo-4-methyl-4-aza-5α-androstane-17β-carboxylate. (See Rasmusson, et al. J. Med. Chem 29, 2298-2315, 1986) was hydrolyzed with 7% KOH in aqueous methanol, followed by an acidic work up to give the corresponding 17β-carboxylic acid.

The acid was readily converted into benzotriazyl-1-yl-3-oxo-4 methyl-4-aza-5α-androstane 17β carboxylate as described in Example 9. The activated ester (the benzotriazoyl derivative) was purified on TLC (4 plates, 20 cm x 20 cm x 20 cm x 1000µm silica gel) eluted with 4:96 (MeOH-CHCl₃). The isolated product was washed with ether to give the active ester m.pt. 198-200°C with decomposition.

### EXAMPLE 11

### Synthesis of 17β (N-1-adamantylcarbamoyl)-4-methyl-4-aza-5α-androstan-3-one

100.0 mg of the 4-methyl-4-aza-benzotriazole derivative prepared as described in Example 10, was dissolved in 20.0 ml CH₂Cl₂. To the clear solution was added 127 mg of 1-adamantamine. The reaction mixture was stirred overnight at R.T./N₂.

Crystallization from EtOAc after filtering the solution through Teflon Acrodisc CR afforded 26.32 mg, m.pt. 210-217°C. The product was further purified on 1.0 g silica gel column (EM silica gel) with 1:1 (acetone-hexane) as eluant to give after recrystallization (ethyl acetate) 21.75 mg of white needles of the above-titled product, m.pt. 203-205°C.

| Anal. Calcd. for C₃₀H₄₆N₂O₂·1.5 H₂O: | | | | | | |
|---|---|---|---|---|---|---|
| | C, | 73.58; | H, | 9.68; | N, | 5.62; |
| Found: | C, | 73.15; | H, | 9.30; | N, | 5.67. |

### EXAMPLE 12

### Diastereomeric Synthesis of 17β(N-exo-2-norbornanylcarbamoyl)-4-aza-5α-androst-1-en-3-one)

100.0 mg of the corresponding 4-H thiopyridyl ester of Example 6 (See Rasmusson et al. J. Med. Chem. Vol. 29, pp. 2298-2315 (1986), was dissolved in 3.0 ml of dry THF under N₂. To the clear solution was added 477 µl of (±) racemic exo-2-aminonorbornane. Allowed the reaction to proceed for 16 hours at R.T./N₂. The reaction mixture was evaporated to dryness in vacuum. The residue was dissolved in chloroform. The organic layer was washed with 2.5 N HCl acid (3 times); 3 times with water; 3 times with saturated NaCl solution, dried over MgSO₄, filtered and evaporated to dryness in vacuum to afford 56.3 mg of a racemic diastereomeric mixture.

The crude product was chromatographed on TLC (2 plates, 20 cm x 20 cm x 500 µm silica gel) eluted with 70:30 (CHCl₃:acetone) to yield 43.4 mg.of the above-titled product. Recrystallization from EtOAc yielded 30 mg product, m.pt 245-245.9°C.
NMR (CDCl₃) confirmed the above structure.
FAB mass spectrum calcd. for C₂₆H₃₈O₂N₂: m/e 411; Found: 411.

| Anal. Calcd. for C₂₆H₃₈O₂N₂.H₂O: | | | | | | |
|---|---|---|---|---|---|---|
| | C, | 72.82; | H, | 9.40; | N, | 6.58. |
| Found: | C, | 73.21; | H, | 9.20; | N, | 6.25. |

### EXAMPLE 13

### Synthesis of 17β(N-1-adamantylmethylcarbamoyl)-4-aza-5α-androst-1-en-3-one

200.0 mg of the thiopyridyl aza steroid, used in Example 12, was suspended in 2.0 ml of dry THF.

To the suspension was added 400 µl of 1-aminomethylene adamantane via syringe at R.T./N₂. After several minutes, a yellow clear solution resulted and after 1/2 hr., precipitation occurred. The reaction was allowed to proceed overnight/N₂. Diluted with CH₂Cl₂, washed with 10% NaOH, two times, then with H₂O two times, followed by 10% HCl (two times), H₂O (two times), and finally two times with satd. NaCl solution.

The organic layer was dried over MgSO₄, filtered, concentrated in vacuo to obtain the product, as shown by NMR, recrystallized from EtOAc, to yield 149.0 mg product, m.pt 255-257°C with decomposition.
FAB Mass Spectrum, Calcd: m/e 464 + 1 = 465: Found 465.

### EXAMPLE 14

### Synthesis of 17β(N-2-adamantylcarbamoyl)-4-aza-5α-androstan-3-one

A mixture of 1.09 grams 17β-(N-2-adamantylcarbamoyl)-4-aza-5α-androst-1-en-3-one (See Example 3 for preparation), 150 ml of ethanol, and 1.0 g. of 30% Pd/C was hydrogenated overnight with shaking under 45 psig. hydrogen pressure. The suspension was filtered to remove catalyst, and evaporated to dryness to yield a grey residue. This was chromatographed by elution on a 200 ml silica gel column with 40:60 acetone/methylene chloride eluant to yield 1.0 g of solid, mp. 294-296°C.

| Anal. Calcd. for C₂₉H₄₄N₂O₂·0.2H₂0 | | | | | | |
|---|---|---|---|---|---|---|
| Calcd. | C, | 76.33; | H, | 9.80; | N, | 6.14 |
| Found | C, | 76.23; | H, | 9.86; | N, | 5.92 |

Mass Spec. Analysis by electron impact showed MW of 452.

### EXAMPLE 15

### Synthesis of 17β-(N-2-adamantylcarbamoyl)-4-aza-4-methyl-5α-androst-1-en-3-one

A suspension of 500 mg of 17β-(N-2-adamantyl-carbamoyl)-4-aza-5α-androst-1-en-3-one, as prepared in Example 3, 10 ml sieve-dried DMF, 140 mg NaH, were heated and stirred at 70°C under a nitrogen atmosphere for 18 hours. Cooled to room temperature and then added 0.4 ml methyl iodide dropwise with stirring which was continued at 50°C for 3 hours. The reaction mixture was then treated by cooling to room temperature, followed by the addition of 15 ml water. The mixture was extracted with 3 x 20 ml of CH₂Cl₂. The organic layers were combined, washed with brine, dried and evaporated to yield a white crystalline residue. Recrystallization from ethyl acetate/CH₂Cl₂ yielded a pure white solid, mp 246-248°C.

| Analysis calculated for C₃₀H₄₄N₂O₂·0.3H₂0 | | | | | | |
|---|---|---|---|---|---|---|
| Calcd. | C, | 76.65; | H, | 9.56; | N, | 5.95 |
| Found | C, | 76.50; | H, | 9.75; | N, | 5.84 |

Mass spectroscopy showed a molecular weight of 464.

### EXAMPLE 16

### Synthesis of 17β-(N-2-adamantylcarbamoyl)-3-oxo-4-methyl-4-aza-5α-androstane

17β-(N-2-adamantylcarbamoyl)-4-methyl-4-aza-androsten-1-en-3-one, (200 mg) as prepared in Example 3, were placed into 25 ml absolute ethanol with 200 mg 30% Pd/C hydrogenation catalyst. The suspension was rocked overnight under 40 psig hydrogen pressure. The suspension was filtered, and the filtrate evaporated to dryness. The residue was recrystallized from hot ethyl acetate to give a white crystalline solid, mp. 113-115°C.

| Calcd. for C₃₂H₅0N₂0₃·0.5 EtOAc | | | | | | |
|---|---|---|---|---|---|---|
| Calcd: | C, | 75.25, | H, | 9.86, | N, | 5.48 |
| Found | C, | 75.07; | H, | 9.52; | N, | 5.28 |

Mass spectroscopy depicted a molecular weight of 466 for the non-solvated molecule.

### EXAMPLE 17

### Synthesis of 17β-(N-methyl-N-2-adamantyl) carbamoyl-4-methyl-4-aza-androst-1-en-3-one

17β-(N-2-adamantyl)carbamoyl-4-aza-androst-1-en-3-one (5.0 g) and 1.5 g sodium hydride in 100 ml dry DMF were stirred under dry nitrogen for 3 hours at 40°C. The reaction was cooled to room temperature and about 4 ml of methyl iodide was added dropwise and allowed to stir at room temperature for one hour. The reaction was cooled in an ice bath and a large excess of about 250 ml, water was added. The aqueous mixture was extracted with CH₂Cl₂ (3 x 100 ml), the organic extracts combined, washed with H₂O, brine, and then evaporated to dryness to yield crude product. The crude product was eluted on an HPLC column (Si gel) with 10/1 acetone/CH₂Cl₂ to yield 2 peaks having retention times of 3 CV(B) and 3.8 CV(A). Peak (A) was analyzed as per the 4-methylaza titled product of Example 15. The second product (B) was analyzed as the 4-methylaza-17β-(N-methyl-N-2-adamantyl/carbamoyl analog, i.e. the titled compound, mp. 163-165.

| Calcd. for C₃₁H₄₆N₂O₂ | | | | | | |
|---|---|---|---|---|---|---|
| Calcd. | C, | 77.77; | H, | 9.68; | N, | 5.85 |
| Found | C, | 77.29; | H, | 9,79; | N, | 5.77 |

Mass spectrometry showed a molecular weight of 478.

### EXAMPLE 18

### Synthesis of 17β-(N-methyl-N-2-adamantylcarbamoyl) 4-aza-4-methyl-androstan-3-one

The crude reaction mixture from Example 17 (4.6 g) was dissolved in 200 ml ethanol and together with 1.0 g 30% Pd/C was hydrogenated under 40-45 Psig a hydrogen atmosphere at room temperature overnight. The mixture was filtered, residue washed with ethanol. The ethanol solution was evaporated to dryness to yield a crude mixture. Recrystallized from CH₂Cl₂/diethyl ether/hexane to yield 800 mg of the pure monomethyl androstane compound of Example 16, mp 113-115°C. Second and third crops were combined with mother liquor and treated by HPLC as in Example 17 to yield the dimethylated title compound, mp 180-182°C.

| Anal. Calcd. for C₃₁H₄₈N₂O₂ | | | | | | |
|---|---|---|---|---|---|---|
| Calcd. | C, | 77.45; | H, | 10.06; | N, | 5.83 |
| Found | C, | 77.26; | H, | 9.87; | N, | 5.82 |

Mass spectrometry showed a molecular weight of 480.

## Claims

1. A compound of the formula: 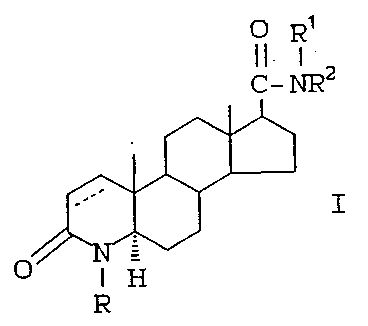 wherein R and R¹ are independently selected from hydrogen, methyl or ethyl; R² is a hydrocarbon radical selected from substituted or unsubstituted 1- or 2-adamantyl, 1- or 2-adamantylmethyl, 1-, 2- or 7-norbornanyl, or 1-, 2- or 7-norbornanylmethyl, provided that when R¹ is hydrogen, then R² does not represent substituted or unsubstituted 1- or 2-adamantyl, or 1- or 2-norbornanyl; wherein
the dotted line represents a double bond which can be present, and pharmaceutically acceptable salts or esters thereof.

2. The compound of claim 1 wherein the 1- or 2-adamantyl or 1- or 2-norbornanyl moieties are substituted with one or more of: C₁-C₄ linear or branched alkyl, nitro, oxo, C₇-C₉ aralkyl, (CH₂)n COOR where n is 0-2 and R is H or linear/branched C₁-C₄ alkyl, CH₂OH, OH, OR where R is C₁-C₄ linear/branched alkyl, halo, COOH, COOR where R is linear/branched C₁-C₄ alkyl,
CONH₂, CH₂NH₂, CH₂NHCOR where R is C₁-C₄ linear/branched alkyl, phenyl, p-nitrophenyl, p-aminophenyl, p-sulfophenyl or cyano.

3. The compound of claim 1 where the 1- or 2-adamantyl or 1- or 2-norbornanyl moieties are unsubstituted.

4. A compound selected from:
17β-(N-1-adamantylmethylcarbamoyl)-4-aza-5α-androst-1-en-3-one;
17β-(N-2-adamantylcarbamoyl)-4-aza-5α-androstan-3-one;
17β-(N-methyl-N-2-adamantylcarbamoyl)-4-methyl-4-aza-androstan-3-one;
17β-(N-2-adamantylcarbamoyl)-4-methyl-4-aza-5α-androstan-3-one;
17β-(N-methyl-N-2-adamantyl)carbamoyl-4-methyl-4-aza-androst-1-en-3-one; and
17β-(N-(3-methyl)-1-adamantyl-carbamoyl)-4-aza-4-methyl-5α-androst-an-3-one.

5. A pharmaceutical composition comprising a pharmaceutically acceptable carrier and a therapeutically effective amount of a compound according to any one of the preceding claims.

6. The use of a compound as claimed in any one of claims 1 to 4 for the manufacture of a medicament for treating the hyperandrogenic conditions of acne vulgaris, seborrhea, female hirsutism, benign prostatic hyperplasia, male pattern baldness or prostatic carcinoma.

7. The use of a compound as claimed in any one of claims 1 to 4 for the manufacture of a medicament for inhibiting both 5α-reductase enzymes 1 and 2.

8. The use of a compound as claimed in any one of claims 1 to 4 for the manufacture of a medicament for inhibiting both 5α-reductase enzymes 1 and 2 in the treatment of the hyperandrogenic conditions of acne vulgaris, seborrhea, female hirsutism, benign prostatic hyperplasia, male pattern baldness or prostatic carcinoma.
